# EUROPEAN PATENT APPLICATION

(11) **EP 2 446 965 A1**
(43) Date of publication of application: **02.05.2012**
(21) Application number: 10189674.4
(22) Date of filing: 02.11.2010
(51) Int. Cl.: B01J 31/02, B01J 31/10, B01J 35/00, B01J 35/02, B01J 37/00, B01J 31/12, C07C 67/03, C08G 77/28

(54) **Process for preparation of supported catalysts and use of the catalyst for the esterification of free fatty acids in vegetable oil**

(71) Applicant: Evonik Degussa GmbH, 45128 Essen (DE)
(72) Inventor: Hausmann, Ralf, 63619 Bad Orb (DE); Fuchs, Manfred, 63755 Alzenau (DE); Röder, Stefan, 36391 Sinntal (DE); Trionfetti, Cristiano, 63457 Hanau (DE)

(57) **Abstract**

Process for preparation of a supported catalyst based on hydroxylated inorganic material selected from the group consisting of silica (SiO₂), alumina (Al₂O₃), titania (TiO₂), zirconia (ZrO₂), lanthanum oxide (La₂O₃) or mixtures thereof,
characterized in that the hydroxylated inorganic material is contacted with organosilicon compounds selected from the group consisting of Formula 1 i.e., [(RO)_{y}Si-[O-(RO)_{y}Si]_{y}-O-Si(RO)_{y}] or
Formula 2 i.e., (RO)_{y}-Si-R¹-S₂₋₄-R¹-Si-(RO)_{y}
with R being alkyl and R¹ being a linear or branched alkylene having from 1 to 5 carbon atoms and y being an integer from 1 to 3.

## Description

The present invention pertains a process for the preparation of a supported catalyst and the use of said supported catalyst for the esterification of free fatty acids in vegetable oil.

### Background of the invention

Biodiesel is a sustainable and renewable fuel produced from various oils, fats and free fatty acids. Today biodiesel is manufactured by transesterification reaction of glycerides with mono-alcohols, most commonly methanol which is used in excess to enhance the mixing of the reactants in order to shift the equilibrium and increase the reaction rate.

The transesterification is typically catalyzed by a base used as homogeneous catalyst, e.g. NaOH, KOH and/or their corresponding alkoxides (see e.g., J. Ruwwe, Chemistry Today, vol. 26 number 1, 2008). Moreover, the manufacturing of biodiesel by transesterification employing heterogeneous base catalysts has been also successfully implemented and commercialized by Axens as described in the US patent 5,908,946. However, in both the cases the use of pretreated feedstock/oils is required in order to
(i) avoid equipment downtime due to soap formation,
   and
(ii) maintain high product's yields (see L.C. Meher et al., Renewable and Sustainable Energy Reviews, vol 10, 3, 2006) .
Thus, the use of the most common and commercially available technology i.e., homogeneous transesterification, is limited to relatively clean vegetable oils containing very low concentration of free fatty acids water and phosphorous, i.e. oils with
(i) a free fatty acid content of less than 0,5 wt%,
(ii)a water content of less than 500 ppm, and
(iii)a phosphorous content of ca. 10 ppm or less.

It is here necessary to stress that the manufacturing costs of biodiesel are dominated by the feedstock costs. Overall factors like investment cost, utilities and labor only play a minor role while the feedstock i.e., soy-bean oil and rapeseed oil represents 80% of the biodiesel costs.

It is generally known that less expensive feedstocks, having high free fatty acids content are available e.g., Jatropha oil, waste vegetable oil (WVO), animal fats. Although biodiesel can be produced from these feedstocks significant processing problems during manufacture exist since the free fatty acids are saponified by the homogeneous alkali catalyst used to transesterify the triglycerides. This may lead to (i) loss of catalyst and (ii) the necessity of additional costly operations such as neutralization, washing, separation, recovery of pure products and generation of contaminated water effluents.

One approach for improving the processing of a cheap feedstock is to remove the free fatty acids from the oil before its transformation to Biodiesel takes place. This is performed at the industrial scale by means of physical and chemical methods. Although physical methods i.e., (i) stripping of fatty acids at high temperature and pressure and (ii) supercritical extraction are generally considered more applicable, their power consumption is extremely high.

The conditions employed for the classical chemical methods seem to be less severe. The methodology aims to esterify the free fatty acids to alkylesters in the presence of an acidic catalyst such as a mineral acid (H₂SO₄, HCl, etc). Thus, the pre-treated oil in which the free fatty acids content is lowerd to ca. 0.5 wt% can then be processed under standard transesterification reaction conditions (see H.N. Basu et al. U.S. Pat. No. 5,525,126).

This pre-treatment step has been successfully demonstrated using sulfuric acid (S. Koona et al., European Pat No. 566047, 1993). Unfortunately as disadvantage, using strong acids, such as H₂SO₄, or p-Me (C6H4) SO3H makes additional neutralization and separation steps necessary. More importantly, the amount of acid required is much higher than the amount of catalyst used to convert the neutral oil to biodiesel. Moreover, the reaction of pretreatment of the vegetable oil also does not go as far to completion as compared to transesterification of the oils and this may lead to a resulting biodiesel product to be out of spec on fatty acids.

In addition, further treatments are needed due to the fact that legal limit of sulfur impurities allowed in diesel are lower than 20 ppm. A feedstock with high sulfur needs to be either blended with a low sulfur feedstock in order to reduce the amount of sulfur in the product or not used at all.

The use of heterogeneous acid catalysts for esterification of fatty acids can definitely improve the existing technology as those catalysts remain active in situ for longer time without any make up steps. In addition, the combination of the use of oil from various sources and heterogeneous catalysts for their pre-treatment can lower costs for making biodiesel. Lewis and Bronsted solid acid catalysts such as metal oxides are suitable to catalyze fatty acid esterification but at relatively high reaction temperatures, higher than 140°C (see G. Rothenberg et al., Energy & Fuels, 22, 598-604, 2008). However, catalysts activity and stability typically decrease when the fatty acids are dissolved in vegetable oils. Therefore the actual disadvantage of solid acid catalysts is that appreciable activity can be recorded only by using, respectively,
(i) temperatures higher than 200°C, where products degradation can take place, and
(ii) a large excess of methanol (ca. 50:1), in order to extract the free fatty acids from the vegetable oil phase.

Under these conditions and in the case of Bronsted acid catalysts, their stability, due to either hydrolysis or leaching of the acid sites leading to catalyst degradation, seems to be the major challenge.

The interaction between inorganic materials and organo silicon compounds is well known in the literature and it was originally the objective of a technology which aims to improve the mixing and the interaction between fillers and rubber compounds for instance for the tires industry (see U.S. Pat. No. 4,514,231). More specifically, the use of silica in combination with alkoxysilanes and/or silanol hindering sulfonic acid groups has been also described in literature as a new class of solid acid (see Lansink-Rotgerink et al. U.S. Pat. No. 5,919,566) and employed for several acid catalyzed reactions (see S. Wieland et al., U.S. Pat. No. 5,922,900). However the challenge here comes from the detachment of the compounds containing sulfur and the low density of active acid sites. These disadvantages are even more pronounced by using supports materials such as cylindrical extrudates or spherical beads due to their poor morphology. Thus, the possibility of making stable and active solid catalysts for fixed bed application results to be another major challenge.

Surfactant-templated mesoporous structured silica based materials have received great attention due to their high surface area and ordered pores. These are obtained by the polymerization of alkoxysilanes around surfactant micelle templates. The properties of these materials can be modified by incorporating functionalized organic groups containing sulfonic acid groups. Although their efficiency for fatty acid esterification has been successfully proven (see, D.R. Radu et al., U.S. Pat. No. 7,122,688) a better way of designing the surface pore structure is the current issue in order to avoid pore blockage and fast deactivation.

The objective of the present invention is thus to provide a process for the production of a catalyst and a catalyst suitable for esterification of free fatty acids in vegetable oils in which the disadvantages of the prior art are at least reduced.

This objective is achieved according to the pertinent claims.

In particular, one feature of the present invention provides a process for preparation of a supported catalyst based on hydroxylated inorganic material selected from the group consisting of silica (SiO₂), alumina (AL₂O₃), titania (TiO₂), zirconia (ZrO₂), lanthanum oxide (La₂O₃) or mixtures thereof,
characterized in that the hydroxylated inorganic material is contacted with organosilicon compounds selected from the group consisting of Formula 1 i.e., [(RO)_{y}Si-[O-(RO)_{y}Si]_{y}-O-Si(RO)_{y}] or
Formula 2 i.e., (RO)_{y}-Si-R¹-S₂₋₄-R¹-Si-(RO)_{y}
with R being alkyl and R¹ being a linear or branched alkylene having from 1 to 6 carbon atoms and y being an integer from 1 to 3

Said process provides a strong acidic supported catalyst based on highly hydroxylated inorganic silica, alumina, titania, zirconia, and lanthanum oxide to be used for fixed bed application.

These hydroxylated inorganic materials are commercially available

The inventive process leads to a functionalized supported catalyst by using grafting technology and having long lifetime and high thermal stability. With respect to grafted molecules high stability is achieved towards hydrolysis. Respectively, (i) catalyst shape and size, (ii) catalyst performance even in presence of soap, and (iii) the unique and low costs manufacturing method will make the materials easily available for the existing biodiesel technology.

The hydroxylated inorganic material is preferably treated with an organosilicon compound to obtain a hydrophobic support needed to protect the catalyst active sites from water molecules deactivation.

The organosilicon compounds employed have a general Formula 1 i.e., [(RO)_{y}Si-[O-(RO)_{y}Si]_{y}-O-Si(RO)_{y}] or the general Formula 2 i.e., (RO)_{y}-Si-R¹-S₂₋₄-R¹-Si-(RO)_{y}.

The starting material of the process according to the invention is hydrophilic, the end product is hydrophobic.

The degree of hydrophobicity can be described by means of the contact angle. It is preferred that the supported catalyst obtained according to the inventive process has a contact angle (θ) higher than 80°, more preferably between 80° and 100°. The contact angle is measured following the so called Standard Sessile Drop Method. In this case, the contact angle is measured by using current-generation systems employing high resolution cameras and software to capture and analyze the contact angle.

R is preferably methyl, ethyl, propyl or butyl, more preferably methyl or ethyl.

R¹ is preferably a linear alkylene having 1 or 2 carbon atoms, i.e. methylene or ethylene. Alkylene means a divalent alkyl radical also often named as alkanediyl.

Most preferably the organosilicon compound according to formula 2 is selected from the group consisting of:

(CH₃CH₂O)₃-Si-CH₂-CH₂-S₄-CH₂-CH₂-Si- (OCH₂CH₃)

(CH3CH₂CH₂O)₃-Si-CH₂-CH₂-S₄-CH₂-CH₂-Si-(OCH₂CH₂CH₃)₃

In the only case of the coating due to the interaction between the compound showed in the Formula 1 and one of inorganic solid supports before mentioned, the surface properties can further be modified by using a different compound of general Formula 3 i.e., [(RO)_{y}Si-R¹-SO₃⁻]ₓ zM.

It is thus preferred in the process according to the invention that the treatment of the hydroxylated inorganic material with an organosilicon compound according to Formula 1 is accompanied or followed by a treatment with an organosilicon compound of Formula 3 [(RO)_{y}Si-R¹-SO₃⁻]ₓ zM with y, R and R¹ having the same meaning as above,
x being an integer from 1 to 4,
M being selected from the group consisting of H⁺ , NH₄⁺ or a metal ion with a valence between 1 and 4 and
z being an integer from 1 to 4 depending on the ionic charge of M such that the ionic charge of the compound of Formula 3 is neutral, e.g. if x is 4 and M is H⁺ then z is 4. If x is 2 and M is a metal ion having a double positive charge Me⁺⁺ then z is 1.

The organosilicon compound according to Formula 1 can be mixed with the organosilicon compound according to Formula 3 and then the mixture can be used for treatment of the hydroxylated inorganic material.

Most preferably the organosilicon compound according to formula 1 is selected from the group consisting of:

[(CH₃CH₂O)₃Si-[O-(CH₃CH₂O)₂Si]₄-O-Si(OCH₂CH₃)₃]

[(CH₃CH₂CH₂O)₃Si-[O-(CH₃CH₂CH₂O)₂Si]₄-O-Si(OCH₂CH₂CH₃)₃]

The organosilicon compound according to formula 3 is most preferably selected from the group consisting of:

[(HO)₃Si-CH₂CH₂-SO₃⁻]H⁺

[(HO)₃Si-CH₂CH₂CH₂-SO₃⁻]H⁺

[(HO)₃Si-CH₂CH₂CH₂CH₂-SO₃⁻]H⁺

The surface modification by using a mixture of organosilicon compound according to Formula 1 and Formula 3 is responsible for an increase of the acidity of the oxides surface. The immobilized acidic functional groups give rise to hybrid particles for catalytic purposes. Their ratio about the compounds in the Formula 1 and Formula 3 can vary between 1:1 and 10:1 and these have to be also tuned based on the amount of solid support used.

Focus in a preferred embodiment is given to the case of silica as inorganic support. The improved stability observed determined is due to the formation of Si-O-Si. The silica surface area is generally pretty high from 100 to 600 m²/g. The surface ability of reacting with a long chain compound described in the formula 1, 2 and 3 can be tested by the adsorption of probe molecules i.e., with carbon chain between 4 and 8 and the detection of a high concentration of hydroxyl groups which tremendously increase the reactivity per m² of material. Such hydroxyl groups act as reactive functional groups and they are stable up to 250°C.

Preferably the silica is mesoporous silica with a pore diameter between 2 and 100 nm. The particle size is preferably starting from 25 µm to 1.2 mm. More preferably the starting carrier materials are non regular spherical particles prepared by precipitation under controlled pH. Such particles are more easily pressed into tablets. The time for the precipitation is strongly influencing the regularity of the particle shape.

In another preferred embodiment the catalyst morphology is dominated by macropores. Such material looks like a sponge and chemical composition reveal the predominant presence of only silicium oxide.

The SEARS number is a measure of the degree of hydroxylation. In order to calculate the SEARS number the silica surface is treated using a solution of NaCl (250g/l). The reaction with surface hydroxyl groups generates HCl which is further titrated using a solution of KOH (0,1 N).

The hydroxylated support used in the process according to the invention preferably has a SEARS number of > 20 [OH]/m².

More preferably the SEARS number lies between 20 and 30. At higher SEARS numbers the material becomes too hydrophobic after impregnation and it is not possible to press the supported catalyst into tablets.

It is further preferred that the ratio CTAB surface area : BET surface area is between 0,90 and 1,00, more preferably between 0,95 and 0,98. The BET surface area is determined by using the Brunauer, Emmett and Teller theory. This consists of physically adsorbing on the surface at 77 K from a monolayer to multilayer of Nitrogen molecules without interaction within absorption layers. The CTAB surface area is calculated following the ASTM method D3765.

Thus, the supported catalyst according to the invention can be prepared from a dispersion in ethanol of the compound showed in the Formula 1 mixed with the one in Formula 3, comprising one or more functional groups sufficiently acidic such as sulfonic acid. The condensation of these two compounds and the interaction with the silica surface takes preferably place at a temperature between 60 and 130°C and strongly modified the properties of the carrier as well as the pore of the silicate.

The impregnation step, i.e. the treatment of the hydroxylated support with the organosilicon compound may be carried out by dipping methods, incipient wetness impregnation or spray impregnation. Overall the mixture catalyst and functionalizing agents is stirred at relatively high temperature. After the impregnation the supported catalyst may then be separated by filtration and washed in water and alcohol to remove the un-reacted species. The optional drying process takes place at a temperature higher than 130°C in order to remove the solvents. Additionally, a curing step at much higher temperatures ranging from 100 to 200°C may also be carried out. The functional groups incorporated on the surface of the pores can be chemically quantified and optionally titrated.

During impregnation hydroxyl groups of the support react with the organosilicon compound to form Si-O-Si bonds and a layer on the support is formed from the organosilicon compound. This layer has an average pore size close to 80 nm giving rise to the formation of a homogeneous sponge like structure. This so called open structure allows a large portion of sulfonic groups from the organosilicon compounds to be easily accessible. The accessibility of the sulfonic groups can be described by the acid number. The acid number is determined by surface titration. The acid number of the thus obtained supported catalyst preferably lies between 80 and 180 mg_{KOH}/gₛₐₘₚₗₑ, preferably between 100 and 130 mg_{KOH}/gₛₐₘₚₗₑ.

The supported catalyst according to the invention can also be prepared by treatment of the hydroxylated inorganic material, preferably a hydroxylated silica, with alkoxysilanes containing mercapto groups, i.e. with organosilicon compounds according to formula 2.

After the treatment the mercapto groups can be converted into an acid group as described below. The advantage of an organosilicon compound of Formula 2 is that the attachment to the hydroxylated support based on a condensation reaction is pretty fast and strong and the resulting materials can show up to a monolayer of mercapto groups on the surface.

The resulting material containing immobilized mercaptoalkyl groups is oxidized to a -SO₃H group by using an oxidizing agent, e.g. H₂O₂ or an acid, e.g. nitric acid. This procedure is preferably carried out in a mixture containing water and ethanol. Usually the hydrogen peroxide dissolved in the alcohol is ca. 3 volume percent. After a suitable time, the suspension is filtered and washed with H₂O and alcohol. The wet material can optionally be suspended one more time in an aqueous solution of inorganic acids, e.g. nitric acid. Finally the materials will be intensively washed with water and dried at elevated temperature in order to yield the resulting supported catalyst. The acid-activated materials are denoted with the suffix -SO₃H.

The shaping of the supported catalyst according to the invention can be carried out before or preferably after impregnation/functionalization of the hydroxylated inorganic support. In the latter case, the acidic functionalized supported catalyst as described before having particle size of several µm should then posses lower hydrophobicity to be pressed together forming tablets of various dimensions between 2,5 and 5 mm. In addition, the functionalized acidic supported catalyst powder can be compacted by using binders with low decomposition temperature, preferably below 200°C. Moreover, extrudates starting from 1 to 3 mm in diameter can also be obtained followed by calcination preferably at temperature not higher than 200°C. The use of a standard compactor is also preferable.

The acidic functionalized supported catalyst produced according to the invention is particularly suitable to esterify the free fatty acids (ffa) contained in vegetable oils to the corresponding fatty acids alkyl esters.

The invention therefore also encompasses a process for esterification of free fatty acids in vegetable oils with an aliphatic alcohol to the corresponding fatty acid alkyl esters at a temperature between 80°C and 160°C, the aliphatic alcohol having a maximum number of carbon atoms of 4, characterized in that the process is carried out using a supported catalyst according to the present invention or a supported catalyst prepared according to the present invention.

Preferably the alcohol is methanol and the alkyl ester is a fatty acid methyl ester.

It is further preferred that the process for esterification of free fatty acids(ffa) in vegetable oils is characterized in that the ratio of free fatty acids in the vegetable oil to aliphatic alcohol ranges from 1:5 to 1:25, more preferably from 1:5 to 1:15, most preferably from 1:5 to 1:10.

The esterification reaction is usually kinetically unfavoured due to higher solubility of fatty acids in triglycerides than in the employed alcohols. Thus, a large excess of alcohol in a ratio of ffa: alcohol of at least 1:40 often also 1:50 or higher is usually a must. The advantage of this invention resides in a catalyst that can selectively convert fatty acids and alcohol, i.e. methanol with a typical ratio ffa: alcohol from 1:5 to 1:25.

The fatty acids are preferably processed continuously. The esterification reaction is carried out a at a temperature between 80°C and 160°C, preferably between 100°C and 120°C. Preferably the esterification is carried out in a fixed bed reactor using a pressure below 10 bars. More preferably the esterification is carried out in a trickle bed reactor. Provided that the supported catalyst is present as coarse particles a fixed bed reactor is preferred for the esterification. When using the supported catalyst as a powder, i.e. with a particle size < 100µm, a fluidized bed reactor is also applicable.

The LHSV (liquid hourly space velocity) for the esterification of free fatty acids may e.g. lie between 0.5 and 1.5 h⁻¹. By liquid hourly space velocity is meant the liquid volume of reactant supplied per hour divided by the volume of catalyst used. The LHSV may be increased by increasing the reaction temperature.

Even high free fatty acids containing samples can easily be treated by the esterification reaction with the supported catalyst produced according to the present invention. The mixture of fatty acid alkyl ester and triglycerides can afterwards be directly sent to the transesterification unit.

Vegetable oils always contain triglycerides and free fatty acids. The free fatty acids have to be transformed into fatty acid alkyl esters before the transesterification of the triglycerides can take place. Since the transesterification is typically catalyzed by a base used as homogeneous catalyst this catalyst would lead to saponification of the free fatty acids. The soap formation can be avoided if the free fatty acids are transformed into fatty acid alkyl ester using a supported catalyst produced according to the present invention.

The present invention is further illustrated by means of the following non-limiting examples. The test of fatty acid esterification has been carried out for carboxylic acids having different carbon chain length i.e., between 5 and 18 and differently saturated, double bonds ranging from 0 to 3.

### Example 1

Ca. 44,0 g of ethyl silicate Wacker TES 40^{®} were mixed with ca. 24,0 g of Si285 (Degussa product [(HO)₃Si-CH₂CH₂CH₂-SO₃⁻]H⁺) and 49,0 g of Ethanol. The mixture was let react for 1 hr at temperature ranging between 20 and 50°C, preferably 20°C. Afterwards, the mixture was let reacting with 30,0 g of precipitated silica powder which was treated at temperatures below 200°C such that hydroxyl groups still exist and that exhibited a SEARS number of 22 and a CTAB:BET surface area ratio of 0,97. The reaction took place at a temperature of 20°C. A paste with specific density and viscosity is kept under stirring for 1 hr to homogenize. The material is then cured at higher temperature between 120 and 150°C for 20 hrs in a vacuum oven, preferably 135°C. The resulting solid is crushed and a milling step is applied. After that the catalyst was washed with water until pH 5 is reached in the water stream and then dried for 6 hrs at temperature between 120 and 150°C, preferably 135°C. The resulting powder undergoes an additional milling step before being ready to be shaped as tablets. The tabletting process is carried out without the use of any binder and 4 mm particles are obtained.

### Example 2

Ca. 44,0 g of ethyl silicate Wacker® TES 40 were mixed with ca. 24,0 g of Si285 and 49,0 g of Ethanol. The mixture was let react for 1 hr at room temperature. Afterwards, the mixture was let reacting with 30,0 g silica powder (Degussa Sipernat^{®}-50) and ca 10 g of hydrate aluminium-magnesium silicate (ACTI-GEL 208) as binder. The coarse particles had a SEARS number of 21 and the ratio CTAB:BET surface area was 0,97. The reaction took place at room temperature. A dense solid was quickly obtained. The material underwent a shaping process using an extruder and extrudates with dimensions between 1 and 3 mm were obtained. Afterwards the extrudates were cured at higher temperature between 120 and 150°C for 20 hrs in a vacuum oven. After that the catalyst was washed with water until pH 5 was reached in the water stream and then dried for 6 hrs at a temperature between 120 and 150°C.

### Example 3

Ca. 44,0 g of ethyl silicate Wacker® TES 40 were mixed with ca. 24,0 g of Si285 and 49,0 g of Ethanol. The mixture was let react for 1 hr at a temperature of 20°C. Afterwards, the mixture was let reacting with 30,0 g of aggregated silica (Sipernat^{®}-50 with Cellulose as binder). The coarse particles had a diameter between 0,8 and 1.2 mm and a SEARS number of 21. The ratio CTAB:BET surface area was 0,94. The reaction took place at room temperature, i.e. at 20°C. The material was then cured at higher temperature between 120 and 150°C for 20 hrs in a vacuum oven. After that the catalyst was washed with water until pH 5 was reached in the water stream and then dried for 6 hrs at temperature between 120 and 150°C. A dense solid is quickly obtained. The material can undergo a shaping process using an extruder adding a silica sol during mixing and extrudates with dimensions between 1 and 3 mm can be obtained. Afterwards the extrudates were cured at higher temperature between 120 and 200°C for 5 hrs. Alternatively the materials without any milling step can be shaped as tablets up to 4 mm diameter.

### Example 4

Ca. 3,6 g of ethyl silicate Wacker Si69® dispersed in 20,0 g of Ethanol is let reacting with 30,0 g of silica powder (Degussa Sipernat^{®}-50) having a SEARS number of 21 and a ratio CTAB:BET surface area of 0,95. The reaction took place at 20°C. A paste with specific density and viscosity was kept under stirring for 1 hr to homogenize. The material was then cured at higher temperature between 120 and 150°C for 5 hrs in the oven in presence of nitrogen gas. Afterwards the catalyst was washed with ethanol, filtered and then dried for 12 hrs at temperature between 50 and 80°C. The material was then suspended in water 1,5 liters containing 50 ml of H₂O₂ (30%) and nitric acid at room temperature. The catalyst powder was then washed with water and dried at 130°C for 7 hrs in presence of nitrogen. The resulting powder underwent an additional milling step before being ready to be shaped as tablets. The tabletting process was carried out without the use of any binder and particles 3 to 5 mm in diameter were obtained.

### Example 5 - Continuous esterification of free fatty acids present in vegetable oil

Ca. 20 ml in volume of catalyst as described in the example 1 was loaded into a fixed bed reactor. The temperature of operation was 120°C and respectively a mixture of soy bean oil and oleic acid (ca. 10wt%) were fed with a LHSV of 1 h⁻¹. The pump was feeding 10 ml/h soy bean oil and ca. 0.2 ml/h of oleic acid. An additional pump was feeding 0,15 ml/h methanol and the pressure installed was ca. 7 bar. Thus, a sort of trickle bed regime was installed. The acid number of the mixture vegetable oil and fatty acids before conversion was ca. 16 mg KOH/g and after catalytic reaction the recorded value was ca. 1 mg KOH/g. The acid number was measured by taking probe sample after specific times and the catalyst can be let run longer under this conditions showing high stability. The catalyst did not need any pretreatment and it was active already using a molar ratio ffa:CH₃OH of 1:6. After one run the vegetable oil is ready to be fed into conventional biodiesel plants producing it via transesterification reaction.

## Claims

1. Process for preparation of a supported catalyst based on hydroxylated inorganic material selected from the group consisting of silica (SiO₂), alumina (AL₂O₃), titania (TiO₂), zirconia (ZrO₂), lanthanum oxide (La₂O₃) or mixtures thereof,
**characterized in that** the hydroxylated inorganic material is contacted with organosilicon compounds selected from the group consisting of Formula 1 i.e., [(RO)_{y}Si-[O-(RO)_{y}Si]_{y}-O-Si(RO)_{y}] or
Formula 2 i.e., (RO)_{y}-Si-R¹-S₂₋₄-R¹-Si-(RO)_{y} with R being alkyl and R¹ being a linear or branched alkylene having from 1 to 6 carbon atoms and y being an integer from 1 to 3.

2. Process according to claim 1, **characterized in that** the treatment of the hydroxylated inorganic material with an organosilicon compound according to Formula 1 is accompanied or followed by a treatment with an organosilicon compound of Formula 3
[(RO)_{y}Si-R¹-SO₃⁻]ₓ zM
with y, R and R¹ having the same meaning as above,
x being an integer from 1 to 4,
M being selected from the group consisting of H⁺ , NH₄⁺ or a metal ion with a valence between 1 and 4 and
z being an integer from 1 to 4 depending on the ionic charge of M such that the ionic charge of the compound of Formula 3 is neutral.

3. Process according to claim 1, **characterized in that** the hydroxylated inorganic material is treated with an organosilicon compound according to Formula 2.

4. Process according to claim 3, **characterized in that** the treated material is oxidized by using an oxidizing agent or an acid.

5. Process according to any one of claims 1 to 3, **characterized in that** the hydroxylated inorganic material is mesoporous silica.

6. Process according to claim any one of claims 1 to 4, **characterized in that** the hydroxylated inorganic material has a SEARS number of 20 or higher.

7. Supported catalyst produced according to any one of claims 1 to 4.

8. Supported catalyst according to claim 7, **characterized in that** the contact angle is 80° or higher.

9. Process for esterification of free fatty acids in vegetable oils with an aliphatic alcohol to the corresponding fatty acid alkyl esters at a temperature between 80°C and 160°C, the aliphatic alcohol having a maximum number of carbon atoms of 4, **characterized in that** the process is carried out using a supported catalyst according to claim 6 or 7
or a supported catalyst prepared according to any one of claims 1 to 5.

10. Process according to claim 5, **characterized in that** the ratio of free fatty acids in the vegetable oil to aliphatic alcohol ranges from 1:5 to 1:25.

11. Process according to claim 8, **characterized in that** the aliphatic alcohol is Methanol.
